# EUROPEAN PATENT APPLICATION

(11) **EP 4 018 925 A1**
(43) Date of publication of application: **29.06.2022**
(21) Application number: 20216422.4
(22) Date of filing: 22.12.2020
(51) Int. Cl.: A61B 5/06, A61B 5/00, A61B 34/20

(54) **DETERMINING THE SHAPE OF AN INTERVENTIONAL DEVICE**

(71) Applicant: Koninklijke Philips N.V., 5656 AG Eindhoven (NL)
(72) Inventor: HENDRIKS, Bernardus Hendrikus Wilhelmus, 5656 AE Eindhoven (NL); WILDEBOER, Rogier Rudolf, 5656 AE Eindhoven (NL); KOLEN, Alexander Franciscus, 5656 AE Eindhoven (NL); KAHLMAN, Josephus Arnoldus Johannes Maria, 5656 AE Eindhoven (NL)
(74) Representative: Philips Intellectual Property & Standards

(57) **Abstract**

A mechanism for determining a shape of an interventional device. A mapping function is used to predict the position of an electrode, mounted on the interventional device, as the interventional device is moved within an anatomical cavity - to thereby form a sequence of predicted positions. The sequence of predicted positions is then processed to predict a shape of the interventional device.

## Description

### FIELD OF THE INVENTION

The present invention relates to the field of monitoring an interventional device, and in particular to determining a current shape of the interventional device.

### BACKGROUND OF THE INVENTION

The medical profession is observing an increasing need to perform interventional treatments, using an interventional device such as a catheter or guidewire, on subjects, and there is an ongoing desire to reduce the chance of (unintentional) damage to a subj ect.

In order to improve the effectiveness, and minimize unintentional damage, of interventional treatment, there is a strong desire to obtain information about the interventional device during an interventional procedure. In particular, information about a position and shape of the interventional device can prove important for aiding the clinician in performing effective interventional treatment.

One recent development that has facilitated the determination of the shape of an interventional device within a patient is a fiber-optic shape determination mechanism, such as those described in Jäckle, Sonja, et al. "Fiber optical shape sensing of flexible instruments for endovascular navigation." International Journal of Computer Assisted Radiology and Surgery 14.12 (2019): 2137-2145. This technology works by monitoring the strain within an optical fiber formed in the interventional device. The strain is monitored by monitoring changes in light reflected by fiber Bragg gratings in the optical fiber.

One disadvantage of the fiber-optic shape determination system is that it requires modifications to the interventional device to incorporate this shape sensing technology, as well as an additional component (a light emitting source) externally to the interventional device.

There is therefore a desire to provide an improved mechanism for determining the shape of an interventional device, e.g. during an interventional treatment, whilst reducing or minimizing any alterations of the interventional device.

### SUMMARY OF THE INVENTION

The invention is defined by the claims.

According to examples in accordance with an aspect of the invention, there is provided a computer-implemented method for determining the shape of an interventional device within a subject, wherein the interventional device comprises an electrode responsive to electric or electromagnetic fields.

The computer-implemented method comprises: obtaining, at an input interface, two or more electrical responses of the electrode of the interventional device to crossing electric or electromagnetic fields induced within the subject; obtaining a sequence of positions of the electrode by processing obtained electrical responses using a mapping function to predict, for each electrical response, a respective position of the electrode of the interventional device within a multidimensional co-ordinate space; and processing the sequence of positions to predict a shape of the interventional device within the subject.

The present disclosure provides a mechanism for determining the shape that a (flexible) interventional device takes during an interventional procedure of a subject. The interventional device comprises, e.g. mounts, one or more electrodes that are responsive (e.g. have a different voltage response) to crossing electric and/or electromagnetic fields induced within the subject. The crossing electric fields facilitates identification of the position of (part of) the interventional device, as an appropriate mapping function is able to predict a position based on the response of the electrode to the crossing electric fields. This is because the response of the electrode will change dependent upon its position within the overlap of the crossing electric fields.

The present invention relies upon a concept of tracking the positions of the interventional devices, and using the tracked positions to determine or predict a shape of the interventional device. This disclosure recognizes that the shape of the interventional device will be defined by the movement of the interventional device within the anatomical structure (i.e. a path taken by the interventional device).

Examples of suitable interventional devices include catheters, sheaths, guidewires and so on. In particular, the interventional device may be an elongate and/or flexible interventional device, whose shape changes as it progresses through anatomical cavities (such as blood vessels, bronchi of the lungs or the like). In particular examples, the interventional device may be an interventional device suitable for being modelled as a continuous structure, e.g. can be modelled as a single continuous line.

The sequence of positions of the electrode is preferably ordered by the time at which each electrical response was (originally) obtained or captured at the interventional device. This embodiment recognizes that the shape of the interventional device may depend upon the order in which the interventional device reaches each position, e.g. how the interventional device moves through the subject. This is because the shape of an interventional device will change based on how it moves through the subject.

Effectively, the position of the electrodes can be logged as a function of time (forming a sequence of positions). This can be performed, for instance, by obtaining a timestamp for each response (the timestamp indicating the time at which the response was first recorded). This approach facilitates accurate reconstruction of the shape of the interventional device, as the shape must be restricted to a shape that can map to the position(s) of the electrodes on the interventional device over time.

The electrode of the interventional device may be located at a tip of the interventional device. Tracking the tip of the interventional device facilitates more accurate identification of how the interventional device needs to adapt to movement within the subject, i.e. how the shape of the interventional device changes based on its positions or movement (as the tip of the interventional device will lead or head up any changes to the shape of the interventional device).

The step of processing the sequence of positions to determine a shape of the interventional device optionally comprises: processing the sequence of positions to determine a path followed by the interventional device; and processing the determined path to predict the shape of the interventional device. This embodiment recognizes an explicit link between the path taken by an interventional device and the shape of the interventional device. In other words, it is recognized that the shape of an interventional device will depend upon the path taken by the interventional device within the subject.

Preferably, the two or more electrical responses includes the most recently obtained electrical response of the electrode. Thus, the determined shape of the interventional device may reflect the most recently available shape of the interventional device. This will more greatly assist a clinician in performing a clinical procedure involving the interventional device.

The step of processing the sequence of positions to determine a shape of the interventional device may comprises: obtaining device information defining one or more characteristics of the interventional device; and processing the sequence of positions and the device information to predict a shape of the interventional device within the subject.

This embodiment recognizes that characteristics (e.g. initial shape, structure, flexibility, material properties, orientation(s) and so on) affect the shape and/or path taken by an interventional device as it moves through a subject. By taking one or more such characteristics into account, a more accurate determination of the shape of the interventional device can be achieved.

Preferably, the device information comprises information responsive to a flexibility of the interventional device. A flexibility, e.g. defining a maximum bending radius or the like, provides important information on the range of possible shapes for the interventional device, i.e. provides boundaries or limits on the possible shape of the interventional device. By taking such characteristics into account, a more accurate prediction of the shape of the interventional device can be achieved.

The computer-implemented method may further comprise a step of displaying, at an output display, a visual representation of the shape of the interventional device. Displaying the shape of the interventional device provides useful clinical information to a user to aid them in the performance of a technical task of maneuvering the interventional device within the subject. In particular, a continued machine-human interaction (by monitoring the shape of the interventional device) provides clinical information that assists in the performance of a technical task of investigating an anatomical cavity.

Preferably, this step is performed by controlling signals at an output interface connected to an output display or other user interface.

In some examples, each position in the sequence of positions is a position of the interventional device relative to an anatomical model of an anatomical cavity. In particular, each position may be in a same co-ordinate system in which the anatomical model of the anatomical cavity is defined.

Optionally, the step of processing the sequence of positions to determine a shape of the interventional device comprises: obtaining the anatomical model of the anatomical cavity; and processing the sequence of positions and the anatomical model to predict a shape of the interventional device within the subject.

It is also recognized that the shape of the interventional device will be restricted by the bounds of the anatomical cavity into which the interventional device is placed. For instance, if positioned in a blood vessel, it can be assumed that the interventional device will not extend outside the bounds of the blood vessel (or may extend by only a predictable amount - e.g. to account for pushing or deformation of the blood vessel).

By using the anatomical model (and by defining the position of the interventional device with respect to the anatomical model), additional constraints, restrictions or bounds can be placed on the possible shape of the interventional device.

The computer-implemented method may further comprise a step of displaying, at an output display, a visual representation of the anatomical model of the anatomical cavity and the interventional device, wherein the display of the interventional device is based on at least the determined shape of the interventional device.

There is also proposed a computer-implemented method of identifying the position of an interventional device with respect to a medical image of the subject. The computer-implemented method comprises: obtaining the medical image of the subject; determining the shape of an interventional device within a subject by performing any previously described computer-implemented method; and processing the medical image and the predicted shape to identify a relative position of the interventional device with respect to the medical image.

There is also proposed a computer program product comprising computer program code means which, when executed on a computing device having a processing system, cause the processing system to perform all of the steps of the method according to any herein described method.

Similarly, there is also proposed a computer-readable (storage) medium comprising instructions which, when executed by a computer or processing system, cause the computer or processing system to carry out (the steps of) any herein described method. There is also proposed a computer-readable data carrier having stored thereon the computer program (product) previously described. There is also proposed a data carrier signal carrying the computer program (product) previously described.

There is also proposed a processing system for determining the shape of an interventional device within a subject, wherein the interventional device comprises an electrode responsive to electric or electromagnetic fields.

The processing system is configured to: obtain, at an input interface, two or more electrical responses of the electrode of the interventional device to crossing electric or electromagnetic fields induced within the subject; obtain a sequence of positions of the electrode by processing obtained electrical responses using a mapping function to predict, for each electrical response, a respective position of the electrode of the interventional device within a multidimensional co-ordinate space; and process the sequence of positions to predict a shape of the interventional device within the subject.

Preferably, the sequence of positions of the electrode is ordered by the time at which each electrical response was obtained. In some examples, the electrode of the interventional device may be located at a tip of the interventional device.

In some examples, the processing system may be configured to display, at an output display, a visual representation of the anatomical model of the anatomical cavity and the interventional device, wherein the display of the interventional device is based on at least the determined shape of the interventional device. The processing system may comprise an output interface for communicating with the output display, e.g. providing output signals, for controlling or defining an operation of the output display.

The processing system may obtain the two or more electrical responses from the interventional device and/or a memory (e.g. storing responses of the electrode(s) of the interventional device).

There is also proposed a processing arrangement comprising the processing system. The processing arrangement may comprise the output display. In some examples, the processing arrangement may comprise the interventional device. In some examples, the processing arrangement may comprise the memory.

These and other aspects of the invention will be apparent from and elucidated with reference to the embodiments) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Figure 1 illustrates positions for a set of external electrodes positioned on a subj ect;
Figure 2 illustrates a co-ordinate system for defining a direction of electric fields generated by the external electrodes;
Figure 3 illustrates a processing arrangement that generates a mapping function and an anatomical model;
Figure 4 illustrates an approach for reconstructing an anatomical model from a point cloud;
Figure 5 is a flowchart illustrating a method;
Figure 6 illustrates scenarios for determining a shape of an interventional device;
Figure 7 is a flowchart illustrating a further method;
Figure 8 illustrates a processing system; and
Figure 9 illustrates a processing arrangement.

### DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides a mechanism for determining a shape of an interventional device. A mapping function is used to predict the position of an electrode, mounted on the interventional device, as the interventional device is moved within an anatomical cavity - to thereby form a sequence of predicted positions. The sequence of predicted positions is then processed to predict a shape of the interventional device.

Disclosed concepts are based on the realization that the shape of the interventional device within an anatomical cavity depends upon the path taken by the interventional device within the anatomical cavity (which path is represented by the sequence of the predicted positions). Processing a sequence of positions thereby facilitates the determination of the structure and shape of the interventional device.

Embodiments may be employed in any suitable interventional device tracking system that employs the electric response of an interventional device to predict a position of the electrode within an anatomical cavity. For instance, embodiments may be employed in dielectric imaging systems.

For the purposes of contextual understanding, the principle and purpose of inducing electric fields within a subject will be hereafter described. The skilled person would appreciate how this approach could be adapted for inducing electromagnetic fields.

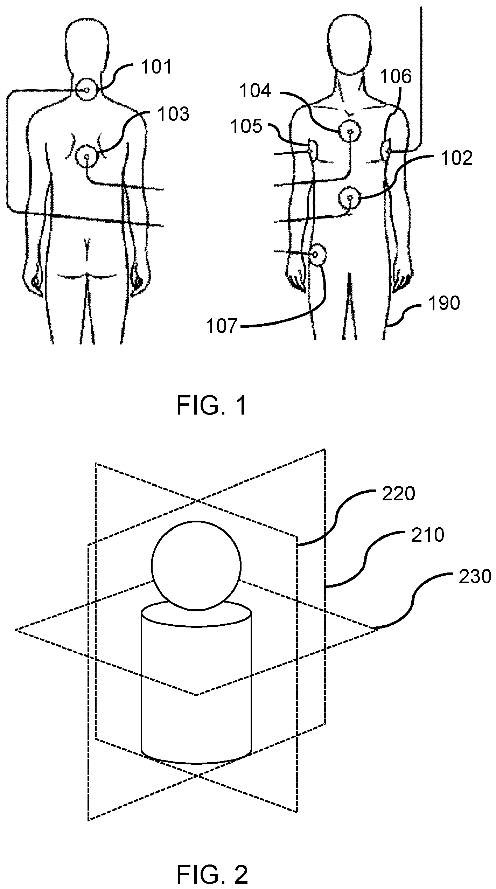

Figure 1 illustrates positions for a set of external electrodes positioned on a subject 190 (i.e. an individual, such as a person or animal). External electrodes are electrodes that are positioned externally to a subject, and are contrasted with internal electrodes that are positionable within the subject.

The illustrated set of external electrodes comprises a first external electrode 101, a second external electrode 102, a third external electrode 103, a fourth external electrode 104, a fifth external electrode 105 and a sixth external electrode 106. The illustrated set further comprises a reference electrode 107, which can act as a "ground" for defining a base/background level of electric field activity in the subject.

An electric signal provided to each external electrode is controlled to thereby define crossing electric fields between the electrodes. In particular, the first 101 and second 102 external electrodes form a first pair of external electrodes ("external electrode pair"), and signals provided to the first external electrode pair can be controlled to induce a first electric field therebetween. The third 103 and fourth 104 external electrodes form a second pair of external electrodes, and signals provided to the second external electrode pair can be controlled to induce a second electric field therebetween. The fifth 105 and sixth 106 external electrodes form a third pair of external electrodes, and signals provided to the third external electrode pair can be controlled to induce a third electric field therebetween.

Electric signals provided to each external electrode, and in particular to each pair of electrodes, can control the frequency and/or magnitude/strength of the crossing electric fields. The electric signals supplied to the electrodes may be controlled by an electric field generator (not illustrated in Figure 1).

The crossing electric fields are usable to track or identify a location of any electrodes positioned within the (overlap of the) crossing electric fields. In particular, a response of an electrode to each electric field will change as a position within the electric field changes (e.g. a distance from a source/sink of the electric field changes). The response of an electrode to the crossing electric fields can be mapped or associated with a particular position within the electric fields.

In other words, an electrical response (which can be alternatively labelled a "measurement") of the internal electrode to the crossing electric fields can be processed to determine a relative position of the internal electrode (e.g. and therefore of any interventional device comprising the internal electrode) within an anatomical cavity of the subject.

In particular, each electric field may be controlled to have a particular/different frequency. This facilitates determination of the relative position of an electrode with respect to each electric field, by assessing the response of the electrode to the particular frequency of the electric field. This information can be used to effectively identify or predict the position of the electrode with respect to a co-ordinate system defined by the electric fields.

One of the external electrode pairs may be omitted, e.g. if only two crossing electric fields are desired (e.g. for performing a 2-dimensional tracking process).

Figure 2 schematically illustrates a co-ordinate system for defining a direction of electric fields generated by the external electrodes. The co-ordinate system defines three cardinal planes 210, 220, 230. The crossing electric fields may, for instance, be optimally positioned when the direction of each electric field is parallel to a respective cardinal place 210, 220, 230.

The intent of the crossing electric fields, e.g. such as those generated using the set illustrated by Figure 1, is to facilitate identification of the position of an electrode (or group of electrodes) with respect to a co-ordinate system, such as that illustrated by Figure 2.

A more complete example of how to track the position of an internal electrode with respect to crossing electric fields (i.e. within the subject), and optionally how to further exploit this information, e.g. for the construction of an anatomical model of an anatomical cavity of the subject, is hereafter described for the purposes of contextual understanding.

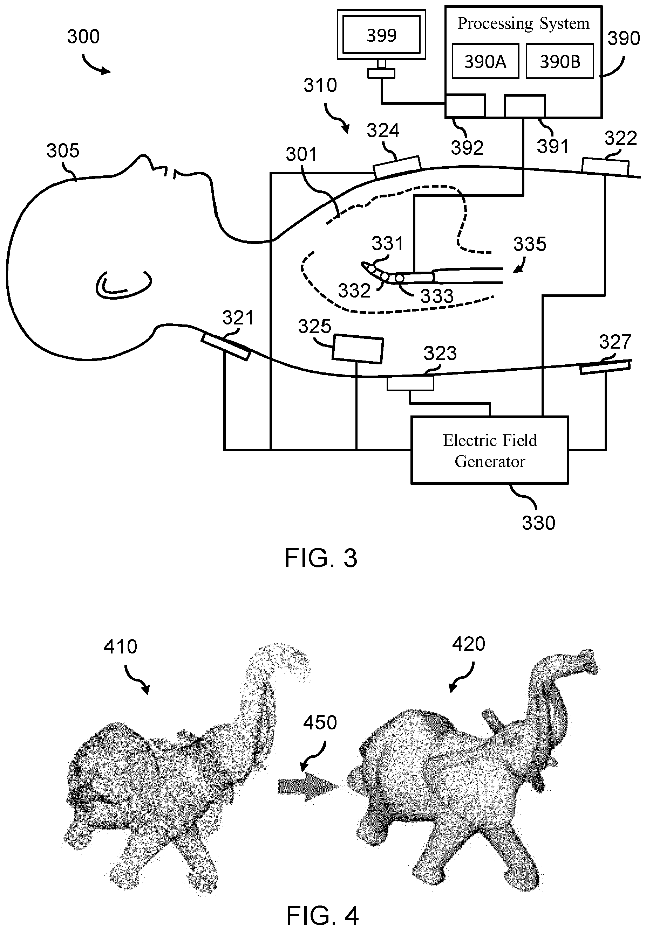

Figure 3 conceptually illustrates a processing arrangement 300 for generating a mapping function for predicting the position of an electrode within an anatomical cavity 301 of a subject 305 (e.g. blood vessels, gastrointestinal tract, the cardiac system and so on). The processing arrangement 300 may further generate an anatomical model of an anatomical cavity within a subject.

In particular, the processing arrangement 300 may be configured to generate an anatomical model (of an anatomical cavity, such as a blood vessel and/or chamber) using a dielectric imaging process.

The processing arrangement 300 comprises an exemplary electric field generating arrangement 310 and a processing system 390. The processing system 390 may contain a mapping function generator 390A (i.e. a "processor") and/or a dielectric imaging processor 390B, although these may be alternatively positioned externally to the processing system 390.

The electric field generating arrangement 310 comprises a set of external electrodes 321, 322, 323, 324, 325, 327 positioned externally with respect to the subject 305 (e.g. as electrode patches provided on a skin of the subject). The set 310 of external electrodes may comprise a plurality of electrode pairs angled with respect to one another (e.g. orthogonal to one another), so that any electric fields generated by the electrode pairs are angled with respect to one another. These electrode pairs may comprise a first electrode pair (formed of a first 321 and second 322 external electrode), a second electrode pair (formed of a third 323 and fourth 324 external electrode) and a third electrode pair (formed of a fifth 325 and sixth (not visible) external electrode). One of more of these electrode pairs may be omitted. The set of external electrodes may also comprise a reference electrode 327. The external electrodes are placable in a similar manner to the set of external electrodes illustrated in Figure 1.

The electric field generating arrangement 310 also comprises an electric field generator 330, which is adapted to control (characteristics of) an electric signal (e.g. voltage and/or current) supplied to each external electrode. In some examples, this may form part of the processing system 390.

The electric field generating arrangement 310 is configured to generate multiple (here: three) crossing (intra-body) electrical fields using the external electrodes. This is performed using appropriate control of the electric signals provided to each external electrode.

In particular, each electrode pair may be appropriately controlled to induce an electric field between each electric pair. Thus, where there are three electrode pairs, three electric fields may be generated. Preferably, the frequency of each generated electric field is controlled to be different, to facilitate increased ease in identifying a relative location of an electrode positioning within the crossing electric fields.

The generated electric fields can be used to define or establish a relative location of an (internal) electrode positioned within the crossing electric fields. In particular, as previously explained, the (electrical) response of an internal electrode to the electric fields changes as the relative position of the internal electrode moves about the subject. This is at least partly because the induced electrical fields' distribution is inherently inhomogeneous due to the different dielectric properties and absorption rates (related to conductivity) of the interrogated tissues. The principle of crossing electric fields thereby facilitates tracking of the relative position of the internal electrode using a processing system 390 that monitors a response of any internal electrodes to the crossing electric fields, e.g. by mapping the (electrical) response of an electrode to a relative position within the subject or using a suitable mapping/transfer function, e.g. the "V2R function" described below to determine the relative position(s) of the electrodes.

By way of further explanation, for the purposes of improved conceptual understanding, if the crossing electric fields are controlled to have a different frequency with respect to one another, then the response of the internal electrode to each frequency could be used to determine a relative distance between from each source/sink of the corresponding electric field. This principle can be used to effectively triangulate the relative position of the internal electrode within the crossing electric fields.

For instance, if there are three external electrode pairs, positioned to emit electric fields (E₁, E₂, E₃) of different frequencies that are angled (e.g. near-orthogonal) with respect to one another, a voltage response (V₁, V₂, V₃) of an internal electrode (identifying a voltage (e.g. between the electrode and the reference electrode or between the electrode and the electrode generating the electric field) at each of these three frequencies) will differ depending upon position within the anatomical cavity.

The skilled person will appreciate that determining the position of internal electrodes 331, 332, 333 also facilitates determining the position, orientation and/or angle of an interventional device 325 that mounts the electrodes. In particular, a positional relationship of the electrodes on the interventional device may be known/predetermined, and used to derive an orientation of the interventional device (e.g. define an axis along which the interventional device lies).

Other forms of response for an internal electrode (e.g. an impedance response or a capacitive response, e.g. indicating change in impedance/capacitance between the internal electrode and an external electrode) will be apparent to the skilled person.

The electric field generator 330 may be configured to control the electric fields, generated using the set of external electrodes, to operate in the frequency range of 30-100 kHz. This frequency range is particularly useful for ensuring penetration of a subject, whilst providing a frequency range that attenuates in human tissue without significant damage/injury to the tissue. The reference electrode serves as an electric reference for all measurements taken by electrodes, e.g. as a reference for the response of the internal electrodes.

The response of two or more internal electrodes mounted upon a single interventional device can be used to construct (and update) the mapping function used to map a response of the internal electrode to a predicted position within the anatomical cavity (e.g. within some Euclidian space).

An exemplary process for generating or constructing a mapping function ("V2R function") using a mapping function generator 390A is hereafter described.

As previously mentioned, the mapping function generator makes use of a plurality of internal electrodes 331, 332, 333 positioned and moved within the anatomical cavity (e.g. electrodes positioned on an interventional device 335 to be inserted into the anatomical cavity). A spatial relationship (e.g. a distance) between each of the internal electrodes may be predetermined and/or known.

The mapping function generator 390A is configured to receive (and optionally provide) signals (e.g. at an input interface 391) from/to the internal electrodes 331, 332, 333 to determine a response of the internal electrodes to the crossing electric fields.

The response of the internal electrodes 331, 332, 333 (e.g. to externally applied electric fields by the external electrodes) is then iteratively recorded for different positions and/or orientations of the interventional device 335, i.e. to obtain a "measurement" from each internal electrode as the interventional device is moved within the cavity 301. The mapping function generator can then repeatedly define/update and apply a mapping function or transfer function ("V2R function") that transforms each recorded response to Euclidian coordinates (R-space), whilst ensuring known properties and/or spatial relationships of the internal electrodes and/or interventional device 335 (e.g. electrode spacing and electrical weight length) as well as a set of other constraints are maintained. For instance, the mapping/transfer function can effectively learn and linearize the distorted relation between the measured responses and the actual position in three dimensions by taking the known inter-electrode distances as a reference. By way of example only, if an internal electrode has a response matching a previously measured response then the relative distance to the responses measured by the other internal electrodes can be determined and used to improve the mapping/transfer function.

In other words, the mapping/transfer function effectively determines or predicts a relative/predicted location of each electrode in a Euclidian and/or multidimensional space or co-ordinate system ("R-space"). In this way, an R-space cloud of points (known Euclidian co-ordinates) can be built up and updated as the internal electrodes are moved within the anatomical cavity. This R-space cloud of points is then iteratively analyzed in order to iteratively update the mapping function to adhere to the known properties of the internal electrodes and/or interventional device, such as a spatial relationship of the internal electrodes.

Another description and/or embodiment of the process for generating the mapping function is disclosed by the European Patent Applications EP 0775466 A2, EP 3568068 A1 and EP 3607879 A1.

The predicted positions of an internal electrode or internal electrodes may also be used to construct an anatomical model of an anatomical cavity (i.e. a cavity in which the interventional device is able to move). This process is called a dielectric imaging process, and may be performed by the dielectric imaging processor 390B.

Broadly, the dielectric imaging processor 390B may build an anatomical model of an anatomical cavity 395 (e.g. a chamber, vessel or void) within a subject by processing an R-space cloud of points. In particular, using an updated R-space cloud of points, such as the R-space cloud of points produced during the construction of the mapping function, a reconstruction algorithm generates an anatomical model of the (investigated part of the) anatomical cavity. The anatomical model may, for example, be a 3D surface that depicts or models the (known bounds of) the anatomical cavity.

The process of reconstructing an anatomical model from a point cloud is conceptually illustrated in Figure 4, which demonstrates a process 450 in which a cloud of R-space points 410 ("point cloud") is transformed into an anatomical model 420. In the illustrated example, this is performed by creating a (3D) surface from the point cloud data, methods of which will be readily apparent to the skilled person.

For example, a point cloud can be converted into a polygon mesh or triangular mesh model (or other surface model) using a surface reconstruction approach. A variety of suitable approaches is discussed in Berger, Matthew, et al. "A survey of surface reconstruction from point clouds." Computer Graphics Forum. Vol. 26. No. 1. 2017, and further mechanisms will be readily apparent to the skilled person.

In this way, the "global field" measurements (i.e. the effect of the electric fields generated by external electrodes on the internal electrodes) can be used to generate a rough anatomical model of the anatomical cavity.

Referring back to Figure 3, more precise identification of the bounds and features of the anatomical model can be performed by monitoring the response of the internal electrodes 331, 332, 333 to local electric fields (e.g. fields generated by other internal electrodes). Thus, in some embodiments, the dielectric imaging processor 390B may also control the internal electrodes 331, 332, 333 to generate an electric field (which can be detected by other internal electrodes). The response of the internal electrodes to local electric fields can be labelled "local field measurements", as compared to the response of the internal electrodes to externally induced electric fields which can be labelled "global field measurements".

For instance, changes in a local electrical field (being an electric field induced between two internal electrodes 331, 332, 333) can indicate the presence or absence of tissue between the two internal electrodes. A response of an internal electrode to a local electric field can therefore be used to identify the presence or absence of tissue, to thereby facilitate tuning or updating of the anatomical model.

In some examples, additional processing of global field measurements (i.e. the responses of the internal electrodes to electric fields induced by the external electrodes) can be performed to improve the precision of the bounds and features of the anatomical model.

For example, regions with inherently marked steep gradients in the electrical field responses can be identified. It is recognized that such regions indicate the bounds of the anatomical cavity and/or other information, e.g. drainage of vessels into or out of a cardiac chamber as well as the valves of a cardiac chamber. These features can thereby be uniquely identified by the system and imaged even without physically visiting them with the interventional device 335.

The combined global and local field measurements enable sophisticated detection and effective handling of inconsistencies and outliers, level of electrode shielding/coverage (e.g. by measuring location inter-correlation), pacing (saturation), as well as physiological drift. Drift can, for example, be detected using a moving window over time and corrected continuously whereby the internal electrode location remains accurate throughout the whole procedure making the system resilient to drift.

The above-provided description of a dielectric imaging process is only an example, and the skilled person would be readily capable of modifying the described process appropriately.

The skilled person will appreciate that the derived mapping/transfer function ("V2R function") can also be used to track a location of one or more internal electrodes with respect to a constructed anatomical model. This could facilitate the generation and display of an anatomical model and an indicator of a current location/position of an interventional device 335 mounting internal electrodes 331, 332, 333 with respect to the anatomical model. Of course, a visual representation of any generated anatomical model and/or determined position may be generated and provided at an output display 399.

The processing system 390 comprises an input interface 391 for receiving signals responsive to the electrical response of the internal electrodes to the electric fields generated by the electric field generating arrangement. The processing system 390 may also comprise an output interface 392 for providing signals for controlling the output display 399.

The present invention proposes to make use of the ability to track the position of an interventional device 335 in order to calculate or determine a shape of the interventional device. In particular, the present invention proposes to take advantage of a recognition that the shape of an interventional device that is maneuvered within an anatomical cavity is dependent upon the positions of the interventional device, and more particularly, on the path taken by an interventional device.

Although an exemplary system using crossing electric fields has been described, the skilled person would readily understand how such a system could be adapted for crossing electromagnetic fields.

For the purposes of this invention, an interventional device is considered to be an elongate and flexible interventional device that can be inserted through anatomical cavities of the subject. Examples of suitable interventional devices include catheters, sheaths, guidewires and so on. The shape of the interventional device changes as it moves through the anatomical cavity, e.g. as it moves through blood vessels, the gastrointestinal tract or the like. This is because the bounds of these blood vessels limit the allowable shape of the interventional device.

In particular, the shape of the interventional device can be modelled as a line that extends through the anatomical cavity. The present invention proposes tracking the position of an electrode mounted on the interventional device in order to determine the line along which the interventional device lies, and therefore define the shape of the interventional device.

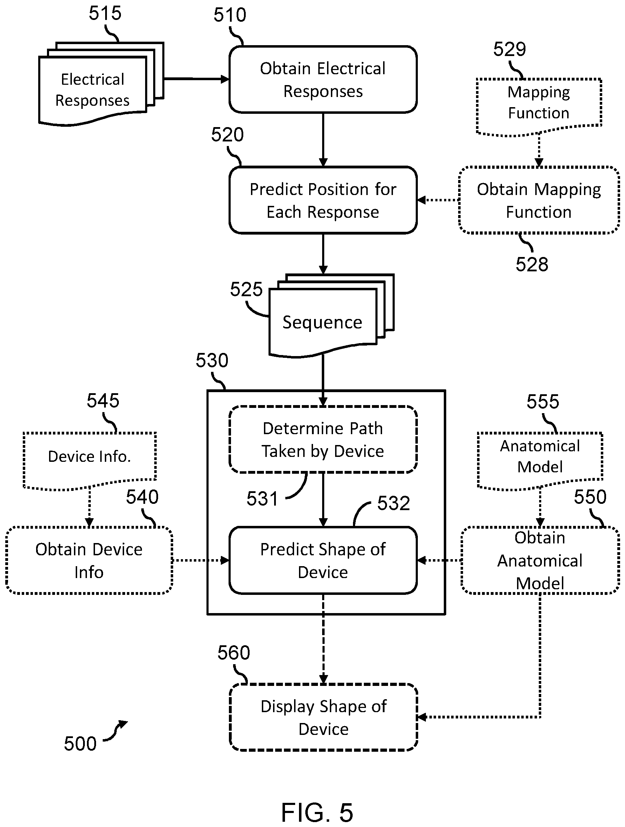

Figure 5 is a flow chart illustrating a method 500 according to an embodiment. The method is carried out by a processing system, such as the processing system 390 described with reference to Figure 3.

The method 500 comprises a step 510 of obtaining, at an input interface (of the processing system), two or more electrical responses of the electrode of the interventional device to crossing electric or electromagnetic fields induced within the subject. The electrical responses may be obtained directly from the interventional device itself (e.g. as illustrated in Figure 3) or from a memory that stores recorded electrical responses of the interventional device.

In some examples, the two or more electrical responses may comprise up to a maximum number of electrical responses. In particular examples, the two or more electrical responses may comprise only the most recently available electrical responses, e.g. the X most recent where X is a predetermined number. Preferably, the two or more electrical responses comprises at least the most recently available electrical response.

The method 500 then performs a step 520 of predicting a position for each response. This is performed by using a mapping function (such as the mapping function previously described) to transform an electrical response to a position within a multidimensional co-ordinate system, i.e. in a multidimensional co-ordinate space. In other words, a position may be a co-ordinate in a multidimensional co-ordinate space. The multidimensional co-ordinate space is defined by the electric fields, and represents a volume or area of the subject.

The step 520 generates a sequence of positions of the electrode. The sequence of positions may be ordered by time at which the electrical responses were originally obtained/captured from the interventional device. This may be determined, for example, by using time stamps that record a time at which an electrical response is captured. In another example, they may be ordered by proximity to one another.

In some examples, the method 500 may comprise a step 528 of obtaining the mapping function 529. The mapping function may be obtained, for example, from a memory and/or a mapping function generator (such as the mapping function generator 590A of Figure 3).

The method 500 may comprise a step 530 of processing the sequence of positions to predict a shape of the interventional device within the subject.

Step 530 is based on the realization that the shape of the interventional device is reflected by the sequence of positions of an electrode mounted on the interventional device. In particular, the shape of the interventional device depends upon how the interventional device is moved within the anatomical cavity, so that the positions of the electrode (mounted thereon) indicate a shape of the interventional device.

Put yet another way, the shape of the interventional device follows a movement of or path taken by the interventional device from one predicted position to another predicted position (e.g. where the possible path may be bound by characteristics of the interventional device and/or the anatomical cavity in which it is placed).

In a particularly preferable example, step 530 comprises using a time-ordered sequence of the predicted positions of the electrodes in order to predict the shape of the interventional device. In other words, it is preferred that the sequence 525 comprises a sequence of predicted positions ordered by time at which the electrode was at the predicted position (i.e. the order in which the corresponding responses were acquired from the interventional device).

This approach recognizes that the order in which the interventional device reaches a particular position influences the shape of the interventional device. In particular, the shape of the interventional device must logically be a shape that results in an ability to place an electrode in the identified positions of the interventional device at different points in time.

By way of example only, in a simple embodiment, the shape of the interventional device may be calculated by defining the shape of the interventional device as a line, curve or other elongate structure that passes through the (e.g. X most recent, where X is a predetermined number) predicted positions of the electrode set out in the sequence 525. Thus, the shape of the interventional device may effectively be defined by the path taken by the interventional device to place an electrode at a particular position.

Smoothing may be applied to the line or curve, e.g. to provide a more realistic shape of the interventional device. Smoothing helps improve the appearance of the overall shape of the interventional device, whilst preserving the accuracy of the position of the tip of the interventional device (i.e. the last known position of the electrode of the interventional device). This provides an operator of the interventional device with an intuitive shape, whilst preserving important information for correct operation and manipulation of the interventional device.

In more complex examples, one or more of the predicted positions of the electrode may be omitted or removed from the sequence used to define the shape of the interventional device. For instance, if a predicted position of an electrode between two other predicted positions in the (time-ordered) sequence means that an unnatural bending of the interventional device must take place (e.g. according to some predetermined parameters or constraints), then this predicted position may be omitted - e.g. as this may indicate an erroneous measurement or (more likely) a change in the shape of the interventional device, e.g. due to retraction of the interventional device.

In some preferred further examples, the latest (i.e. most recently captured) predicted position may not be omitted (i.e. cannot be omitted) from the sequence used to define the shape of the interventional device.

In some examples, step 530 comprises a step 531 of determining a path taken by the interventional device and a step 532 of predicting the shape of the interventional device based on the determined path. The path can be determined by identifying the most recent positions of the electrode mounted on the interventional device. This embodiment recognizes that the path taken by an interventional device defines the shape of the interventional device.

In a simple example, the shape of the interventional device may be defined to match the path of the interventional device. In a more complex, but more accurate, example, the shape of the interventional device may be defined to match the path of the interventional device excepting where the path doubles back on itself, undergoes a bend beyond the bending capability of the interventional device (e.g. as the interventional device explores one channel before retreating) and/or exceeds the bounds of the anatomical cavity.

In order to facilitate exclusion or omission of one or more of the predicted positions in the sequence used to define the shape of the interventional device, the method 500 may comprise a step 540 and/or a step 550.

Step 540 comprises obtaining device information 545 defining one or more characteristics of the interventional device, e.g. information defining a flexibility and/or maximum bending radius of the interventional device. The device information can be used to decide whether a movement between predicted positions is plausible (for an interventional device), e.g. without retreating or withdrawing the interventional device between predicted positions. Thus, the device information may include any characteristic that defines or indicates a maximum plausible/possible bending of the interventional device.

The device information 545 may be obtained from any suitable system, such as a memory or from a user input at a user interface.

Step 550 comprises obtaining an anatomical model 555 of the anatomical cavity. The bounds of the anatomical cavity (which are predicted by the model) will define constraints on the path that an interventional device is able to take, i.e. and thereby provide constraints on a possible shape for the interventional device. The anatomical model is defined in a same co-ordinate space as the predicted positions.

The anatomical model 555 may be obtained from any suitable system, such as a memory or from the dielectric imaging processor 390B (Figure 3). Other suitable systems include systems that generate an anatomical model from other medical images (e.g. from 3D/2D X-ray/ultrasound/MR images. In some examples, an anatomical model generated using a dielectric imaging process is adapted/adjusted based on information contained in one or more other medical images.

Thus, one or more characteristics of the interventional device and the anatomical model (of the anatomical cavity) provide constraints for the possible shape of the interventional device.

In some examples, these two constraints can be used to determine or predict whether an impossible or implausible bend in the interventional device is required to cause the shape of the interventional device to include all of the identified positions (without the interventional device being present outside of the anatomical cavity - whose bounds are indicated by the anatomical model). If an impossible/implausible bend is identified, a predicted position that is the cause of this impossible/implausible bend may be omitted from the sequence used to calculate the shape of the interventional device.

In particular, the excluded predicted position may be the predicted position temporally between two other predicted positions that, together with the predicted position, create an impossible/implausible bend.

In some examples, the device information comprises an orientation and/or angle of the interventional device. Thus, the one or more characteristics may include an orientation and/or angle of the interventional device. It has previously been described how the orientation of an interventional device can be determined based on the response of two or more electrodes mounted on the interventional device.

The orientation of the interventional device can aid in predicting the shape of the interventional device.

In some examples, the device information may comprise an orientation and/or angle of the interventional device at each predicted position of the sequence of positions. This embodiment facilitates improved prediction of the shape of the interventional device, as it can be used to improve a predictive accuracy of the direction in which the interventional device moves between different positions (which defines the shape of the interventional device). For instance, it can be predicted that a device will initially move in a direction in which the interventional device is oriented when moving from one position to another position. This understanding can be used to improve the prediction of the shape of the interventional device.

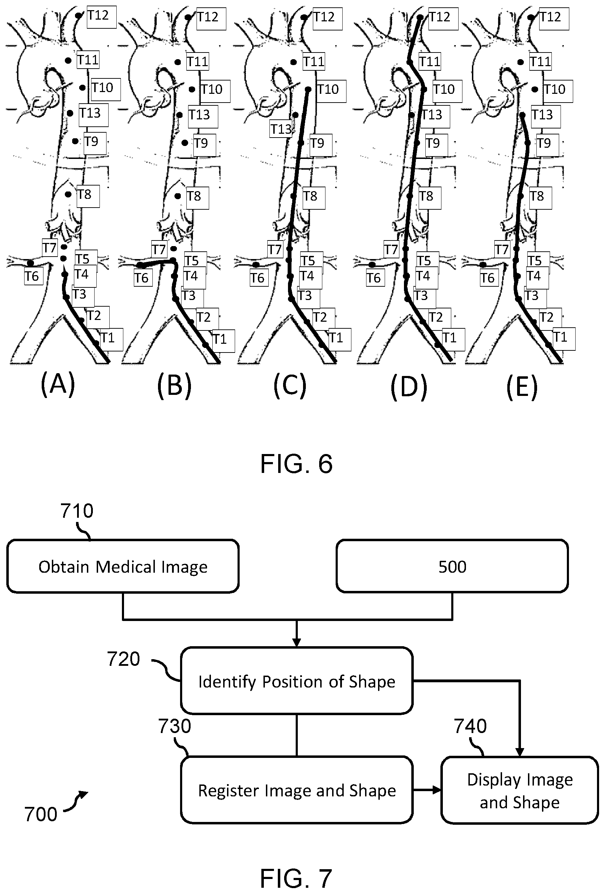

Figure 6 conceptually illustrates the principle of determining a shape of the interventional device 635 in a working example. Here, the interventional device is a catheter inserted through a blood vessel.

In particular, Figure 6 provides a number of diagrams (A) to (E) that demonstrate a predicted shape of the interventional device for different sequences of predicted positions. The predicted shapes are determined using a herein described approach.

The (single) electrode mounted on the interventional device is located at a tip of the interventional device (i.e. a leading tip of the interventional device). This is a particularly advantageous position for an electrode, as it more closely aligns with the path or track followed by the interventional device.

For each diagram, the predicted positions of the electrode are stored as a sequence of predicted positions, the sequence being ordered as a function of time (at which the responses for predicting the positions were originally obtained). The possible positions that can form a sequence are illustrated in the Figure as positions T1 to T13, T1 representing the earliest obtained response and T13 representing the latest. Different diagrams represent a shape constructed from sequences formed from different combinations (e.g. subsets) of the positions T1 to T13 (but which are still time ordered).

Diagram (A) illustrates a first scenario in which the shape is determined using a sequence formed of only positions T1 to T4 (e.g. in a scenario in which T4 is the latest obtained predicted position). The shape of the interventional device is configured to encompass or overlay all of the predicted positions T1 to T4, as there is no implausible/impossible bending of the interventional device required (as determined from device information of the interventional device) to overlay the predicted positions.

Diagram (B) illustrates a second scenario in which the shape is determined using a sequence formed of only positions T1 to T6 (e.g. in a scenario in which T6 is the latest obtained predicted position). The shape of the interventional device is also configured to encompass or overlay all of the predicted positions T1 to T6, as there is no implausible/impossible bending of the interventional device required (as determined from characteristics (of the device information) of the interventional device) to overlay the predicted positions.

Diagram (C) illustrates a third scenario in which the shape is determined using a sequence formed of only positions T1 to T10. In this scenario, there would be an unacceptable/implausible/impossible bending of the interventional device in order to link between positions T5, T6 and T7 - based on known parameters or characteristics of the interventional device and in order to keep the intervention device within the bounds of the anatomical cavity (e.g. as indicated by the anatomical model). This indicates that one or more of these positions should be omitted (from the shape construction process) to avoid this unacceptable bending. In particular, position T6 is not included in the shape construction.

Diagram (D) illustrates a fourth scenario in which the shape is determined using a sequence formed of only positions T1 to T12. The construction of the interventional device is similar to the approach of Diagram (C), but is extended slightly further to account for the additional positions T11 and T12.

Diagram (E) illustrates a fifth scenario in which the shape is determined using a sequence formed of all the positions T1 to T13. The position T13 indicates that the interventional device has been retracted to the position of T13. This can be inferred by the knowledge that a line passing through T11, T12 and T13 would introduce an unacceptable/implausible bend in the interventional device, meaning that positions which cause the unacceptable/implausible bend should be omitted. Thus, a shape of the interventional device can be constructed by omitting positions T10, T11 and T12 (all of which would cause this unacceptable bending to reach T13).

From the foregoing examples, it will be apparent that a shape of an interventional device may be determined by defining a line (e.g. in the multidimensional co-ordinate space) that sequentially connects the predicted positions of a sequence of positions, but optionally omitting one or more of the predicted positions from the sequence of positions (used to define the shape) if connecting that predicted position with its neighbors would contravene constraints set by the one or more characteristics contained in the device information of the interventional device (e.g. a maximum bending radius and/or flexibility) and/or the anatomical model (which defines the bounds of the anatomical cavity - thereby identifying locations at which the interventional device can and cannot exist).

Turning back to Figure 5, the method 500 may further comprise a step 560 of displaying a shape of the interventional device. In particular, step 560 may comprise providing a visual representation of the interventional device, the shape of the visual representation being based on the determined shape of the interventional device.

In particular, step 560 may comprise providing output signals at an output interface of the processing system that (if provided to an output display) would control the output display to display a determined shape of the interventional device.

Step 560 may also comprise displaying a visual representation of the anatomical model (e.g. obtained in step 550). The displayed shape of the interventional device may overlay the displayed anatomical model, so that the relative shape and position of the interventional device within respect to the anatomical cavity (represented by the anatomical model) can be understood. This can be achieved by positioning and sizing the displayed shape to overlay the representation of the anatomical model based on the determined positions used to generate the shape (which positions are in a same co-ordinate system as the anatomical model).

In some examples, the visual representation of the shape of the interventional device may further include known characteristics or features of the interventional device. For instance, if the interventional device comprises a balloon catheter, the visual representation of the interventional device may provide a visual representation of the balloon catheter at the predicted position indicative of the most recently obtained or determined position for the interventional device (i.e. at the tip of the interventional device). As another example, if a diameter or external shape of the interventional device is known, the visual representation of the interventional device may be configured accordingly.

Figure 7 illustrates a method 700 according to a further embodiment of the invention. The method 700 is a computer-implemented method for identifying the position of an interventional device with respect to a medical image of the subject.

The method 700 comprises a step 710 of obtaining the medical image of the subject. The medical image may be any suitable medical image of the subject which contains the anatomical cavity (in which the interventional device is placed). Suitable examples of medical images include an X-ray image, a CT image, an ultrasound image or an MR image. The medical image may be a 2D image or a 3D image, e.g. constructed from a plurality of 2D medical images. In some examples, the medical image may be an image generated from a dielectric imaging process, such as that described with reference to Figures 3 and 4.

The medical image may be obtained from any suitable system, such as a memory or image generator (e.g. an X-ray scanner, a CT scanner or an MRI machine).

The method 700 also performs the process 500, previously described with reference to Figures 5 and 6, for determining the shape of an interventional device within the subject.

The method then performs a step 720 of processing the medical image and the predicted shape to identify a relative position of the interventional device with respect to the medical image. The shape of an anatomical cavity in the medical image will be patient specific.

This disclosure recognizes that, for any given subject, there are (near-)unique possible positions for the interventional device so that the determined shape of the interventional device matches the shape of the anatomical cavity in the medical image. For instance, a blood vessel (e.g. vascular pathway) may have a near unique shape, so that it may only be possible to position an interventional device having a particular shape at a certain location within the blood vessel. Another example are the airways of the lung that provide a bronchi pathway or the structure of a tract in the gastrointestinal system.

The medical image can therefore be processed to identify the part representing the anatomical cavity in which the interventional device having the determined shape is able to be positioned. This approach facilitates registration of the shape of the interventional device (derived from predicted positions of an electrode on the interventional device) with a medical image.

In some examples, the method may further comprise a step 730 of registering the anatomical model and the medical image together. This can be achieved if the anatomical model and the interventional shape are defined with respect to a same co-ordinate system, so that registering the interventional shape and the medical image with respect to one another also facilitates the registration of the anatomical model and the medical image.

The method 700 may comprise a step 740 of displaying the (co-registered) medical image and the shape of the interventional device, e.g. with a visual representation of the shape of the interventional device overlaying the medical image at the determined location. This may be performed by controlling signals provided to an output interface of a processing system.

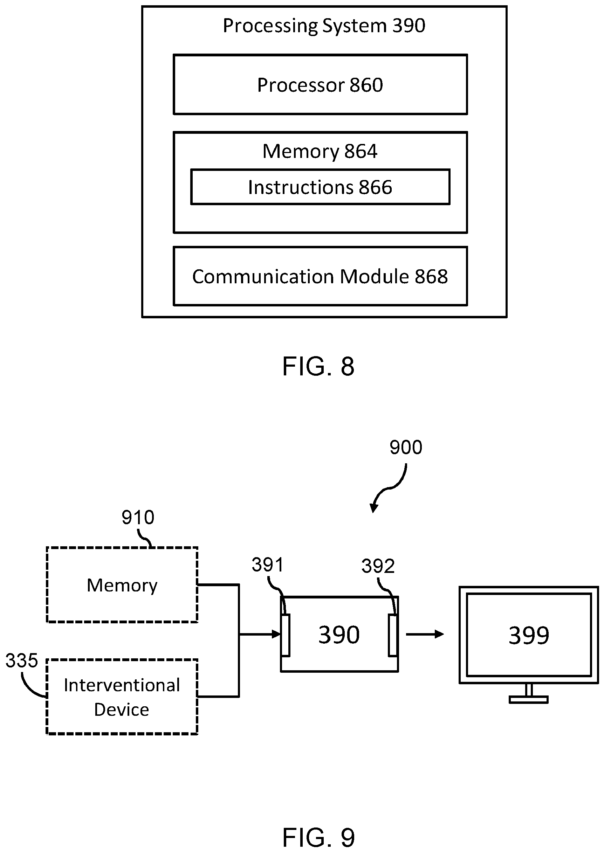

Figure 8 is a schematic diagram of a processing system 390, according to embodiments of the present disclosure. The processing system 390 is configured to carry out a method according to an embodiment, such as the method 500 described with reference to Figure 5.

The processing system 390 may also be configured to generate a mapping function and/or perform a dielectric imaging process, such as those previously described with reference to Figures 3 and 4.

As shown, the processing system 390 may include a (data) processor 860, a memory 864, and a communication module 868. These elements may be in direct or indirect communication with each other, for example via one or more buses.

The processor 860 may include a central processing unit (CPU), a digital signal processor (DSP), an ASIC, a controller, an FPGA, another hardware device, a firmware device, or any combination thereof configured to perform the operations described herein. The processor 860 may also be implemented as a combination of computing devices, e.g., a combination of a DSP and a microprocessor, a plurality of microprocessors, one or more microprocessors in conjunction with a DSP core, or any other such configuration. In some embodiments, the processor is a distributed processing system, e.g. formed of a set of distributed processors.

The memory 864 may include a cache memory (e.g., a cache memory of the processor 860), random access memory (RAM), magnetoresistive RAM (MRAM), read-only memory (ROM), programmable read-only memory (PROM), erasable programmable read only memory (EPROM), electrically erasable programmable read only memory (EEPROM), flash memory, solid state memory device, hard disk drives, other forms of volatile and nonvolatile memory, or a combination of different types of memory. In an embodiment, the memory 864 includes a non-transitory computer-readable medium. The non-transitory computer-readable medium may store instructions. For example, the memory 864, or non-transitory computer-readable medium may have program code recorded thereon, the program code including instructions for causing the processing system 390, or one or more components of the processing system 390, particularly the processor 860, to perform the operations described herein. For example, the processing system 390 can execute operations of the method 500 and/or a method for generating a mapping function and/or performing a dielectric imaging process (as described with reference to Figures 3 and 4).

Instructions 866 may also be referred to as code or program code. The terms "instructions" and "code" should be interpreted broadly to include any type of computer-readable statement(s). For example, the terms "instructions" and "code" may refer to one or more programs, routines, sub-routines, functions, procedures, etc. "Instructions" and "code" may include a single computer-readable statement or many computer-readable statements. The memory 864, with the code recorded thereon, may be referred to as a computer program product.

The communication module 868 can include any electronic circuitry and/or logic circuitry to facilitate direct or indirect communication of data between the processing system 390, the penetration device and/or the user interface (or other further device). In that regard, the communication module 868 can be an input/output (I/O) device. In some instances, the communication module 868 facilitates direct or indirect communication between various elements of the processing system 390 and/or the processing arrangement (Figure 9).

Figure 9 illustrates a processing arrangement 900 according to an embodiment of the invention, which illustrates some optional elements. The processing arrangement 900 comprises a processing system 390, which may be embodied as previously described.

The processing arrangement may further comprise an output display 399. The processing system may be configured to control the output display to display a visual representation of the determined shape of the interventional device and optionally the anatomical model. This control may be performed using an output interface 392 of the processing system 390. The visual representation of the determined shape may be positioned with respect to the anatomical model, e.g. to indicate a predicted shape of the interventional shape within the anatomical cavity represented by the anatomical model.

The processing system 390 may be adapted to obtain the responses of the one or more electrodes of the interventional device from a memory 910 and/or the interventional device 335, e.g. at an input interface 391. The processing arrangement 900 may comprise the memory and/or the interventional device 335 as appropriate.

The memory 910 may also provide the device information of the interventional device (although these could be obtained from another source, such as a user interface or an external server, e.g. contactable over a communication channel such as internet or a LAN connection) and/or the anatomical model, if this is generated by a separate processor (although the anatomical model could be obtained from another source, such as the separate processor, or generated by the processing system 390 itself).

It will be understood that disclosed methods are preferably computer-implemented methods. As such, there is also proposed the concept of a computer program comprising computer program code for implementing any described method when said program is run on a processing system, such as a computer or a set of distributed processors.

Different portions, lines or blocks of code of a computer program according to an embodiment may be executed by a processing system or computer to perform any herein described method. In some alternative implementations, the functions noted in the block diagram(s) or flow chart(s) may occur out of the order noted in the Figures. For example, two blocks shown in succession may, in fact, be executed substantially concurrently, or the blocks may sometimes be executed in the reverse order, depending upon the functionality involved.

The present disclosure proposes a computer program (product) comprising instructions which, when the program is executed by a computer or processing system, cause the computer or processing system to carry out (the steps of) any herein described method. The computer program (product) may be stored on a non-transitory computer readable medium.

Similarly, there is also proposed a computer-readable (storage) medium comprising instructions which, when executed by a computer or processing system, cause the computer or processing system to carry out (the steps of) any herein described method. There is also proposed computer-readable data carrier having stored thereon the computer program (product) previously described. There is also proposed a data carrier signal carrying the computer program (product) previously described.

The skilled person would be readily capable of developing a processing system for carrying out any herein described method. Thus, each step of a flow chart may represent a different action performed by a processing system, and may be performed by a respective module of the processing system.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage.

A single processor or other unit may fulfill the functions of several items recited in the claims. A computer program may be stored/distributed on a suitable medium, such as an optical storage medium or a solid-state medium supplied together with or as part of other hardware, but may also be distributed in other forms, such as via the Internet or other wired or wireless telecommunication systems.

If the term "adapted to" is used in the claims or description, it is noted the term "adapted to" is intended to be equivalent to the term "configured to". Any reference signs in the claims should not be construed as limiting the scope.

## Claims

1. A computer-implemented method (500) for determining the shape of an interventional device (335) within a subject (190, 305), wherein the interventional device comprises an electrode (331, 332, 333) responsive to electric or electromagnetic fields, the computer-implemented method comprising:
obtaining (510), at an input interface (391), two or more electrical responses (515) of the electrode of the interventional device to crossing electric or electromagnetic fields induced within the subject;
obtaining (520) a sequence (525) of positions (T1, T2, T3, T4, T5, T6, T7, T8, T9, T10, T11, T12, T13) of the electrode by processing obtained electrical responses using a mapping function (529) to predict, for each electrical response, a respective position of the electrode of the interventional device within a multidimensional co-ordinate space; and
processing (530) the sequence of positions to predict a shape of the interventional device within the subject.

2. The computer-implemented method (500) of claim 1, wherein the sequence of positions of the electrode is ordered by the time at which each electrical response was obtained.

3. The computer-implemented method (500) of any of claims 1 or 2, wherein the electrode (331) of the interventional device is located at a tip of the interventional device.

4. The computer-implemented method (500) of any of claims 1 to 3, wherein the step (530) of processing the sequence of positions to determine a shape of the interventional device comprises:
processing (531) the sequence of positions to determine a path followed by the interventional device; and
processing (532) the determined path to predict the shape of the interventional device.

5. The computer-implemented method (500) of any of claims 1 to 4, wherein the two or more electrical responses includes the most recently obtained electrical response of the electrode.

6. The computer-implemented method (500) of any of claims 1 to 5, wherein the step (530) of processing the sequence of positions to determine a shape of the interventional device comprises:
obtaining (540) device information (545) defining one or more characteristics of the interventional device; and
processing (532) the sequence of positions and the device information to predict a shape of the interventional device within the subject.

7. The computer-implemented method (500) of claim 6, wherein the device information comprises information responsive to a flexibility of the interventional device.

8. The computer-implemented method (500) of any of claims 1 to 7, further comprising a step (560) of displaying, at an output display, a visual representation of the shape of the interventional device.

9. The computer-implemented method (500) of any of claims 1 to 8, wherein each position in the sequence of positions is a position of the interventional device relative to an anatomical model (555) of an anatomical cavity.

10. The computer-implemented method (500) of claim 9, wherein the step (530) of processing the sequence of positions to determine a shape of the interventional device comprises:
obtaining (550) the anatomical model (555) of the anatomical cavity; and
processing (532) the sequence of positions and the anatomical model to predict a shape of the interventional device within the subject.

11. The computer-implemented method (500) of any of claims 9 or 10, further comprising a step (560) of displaying, at an output display, a visual representation of the anatomical model of the anatomical cavity and the interventional device, wherein the display of the interventional device is based on at least the determined shape of the interventional device.

12. A computer-implemented method (700) of identifying the position of an interventional device with respect to a medical image of the subject, the computer-implemented method comprising
obtaining (710) the medical image of the subject;
determining (500) the shape of an interventional device within a subject by performing the computer-implemented method of any of claims 1 to 11; and
processing (720) the medical image and the predicted shape to identify a relative position of the interventional device with respect to the medical image.

13. A computer program product comprising computer program code means which, when executed on a computing device having a processing system, cause the processing system to perform all of the steps of the method according to any of claims 1 to 12.

14. A processing system (390) for determining the shape of an interventional device (335) within a subject (190, 305), wherein the interventional device comprises an electrode (331, 332, 333) responsive to electric or electromagnetic fields, the processing system being configured to:
obtain (510), at an input interface (391), two or more electrical responses (515) of the electrode of the interventional device to crossing electric or electromagnetic fields induced within the subject;
obtain (520) a sequence (525) of positions (T1, T2, T3, T4, T5, T6, T7, T8, T9, T10, T11, T12, T13) of the electrode by processing obtained electrical responses using a mapping function (529) to predict, for each electrical response, a respective position of the electrode of the interventional device within a multidimensional co-ordinate space; and
process (530) the sequence of positions to predict a shape of the interventional device within the subject.

15. The processing system (390) of claim 14, wherein the sequence (525) of positions of the electrode is ordered by the time at which each electrical response was obtained.
